(19) Europäisches Patentamt
European Patent Office
Office européen des brevets

(11) Veröffentlichungsnummer: **0 429 954 A1**

# (12) EUROPÄISCHE PATENTANMELDUNG

(21) Anmeldenummer: **90121784.4**

(22) Anmeldetag: **14.11.90**

(51) Int. Cl.5: **C07C 39/15**, C07C 37/70, G03C 1/52

(30) Priorität: **23.11.89 DE 3938763**

(43) Veröffentlichungstag der Anmeldung: **05.06.91 Patentblatt 91/23**

(84) Benannte Vertragsstaaten: **DE FR GB Patentblatt**

(71) Anmelder: **HOECHST AKTIENGESELLSCHAFT**
**Postfach 80 03 20**
**W-6230 Frankfurt am Main 80(DE)**

(72) Erfinder: **Siegel, Herbert, Dr. Dipl.-Chem.**
**Haneckstrasse 34**
**W-6238 Hofheim(DE)**
Erfinder: **Lutz, Walter, Dr. Dipl.-Chem.**
**Im Gehren 45 1**
**W-6501 Budenheim(DE)**
Erfinder: **Erdmann, Fritz, Dr. Dipl.-Chem.**
**Am Steinberg 3**
**W-6228 Eltville-Martinstal(DE)**
Erfinder: **Simon, Ulrich, Dr. Dipl.-Chem.**
**Fontanestrasse 41**
**W-6500 Mainz-Lerchenberg(DE)**

## (54) Gemisch überwiegend mehrkerniger polyfunktioneller Phenole und seine Verwendung.

(57) Die Erfindung betrifft ein Gemisch überwiegend mehrkerniger polyfunktioneller Phenole, hergestellt durch Lösen des bei der Resorcinherstellung anfallenden Destillationsrückstands in polaren organischen Lösungsmitteln und/oder Wasser sowie Reinigung der erhaltenen Lösung durch Aktivkohlefiltration gegebenenfalls unter Zusatz von Filterhilfsmitteln, vorzugsweise unter Zusatz von 5 - 20 Gewichtsprozent Resorcin, bezogen auf den eingesetzten Destillationsrückstand.

Das Gemisches wird als Kupplungskomponente zur Herstellung von lichtempfindlichen Diazotypiematerialien, insbesondere in Zweikomponenten-Diazotypiematerialien verwendet.

Das Gemisch wird aus dem Abfall der Resorcindarstellung erhalten und wird als Kupplungskomponente in Diazotypiematerialien verwendet.

EP 0 429 954 A1

## GEMISCH ÜBERWIEGEND MEHRKERNIGER POLYFUNKTIONELLER PHENOLE UND SEINE VERWENDUNG

Die Erfindung betrifft ein Gemisch überwiegend mehrkerniger polyfunktioneller Phenole, erhalten aus dem Destillationsrückstand der Resorcinherstellung und die Verwendung des Gemisches als Kupplungskomponente in lichtempfindlichen Diazotypiematerialien.

Es ist bekannt, daß Mono- und Polyhydroxydiphenyle als Kupplungskomponenten in Diazotypiematerialien eingesetzt werden (DE-A 15 97 617, entsprechend GB-PS 1 226 615). Bei der Kupplung mit Diazoniumsalzen ergeben sich gelbbraune, rotbraune oder violett-schwarze Farbstoffe. Die Verwendung von 2-Hydroxydiphenyl, 3,3′,5-Trihydroxydiphenyl, 2,2′,4,4′-Tetrahydroxydiphenyl, oder 2,3′,4,5′,6-Pentahydroxydiphenyl als Braunkuppler in Diazotypieprozessen ist auch in J. Kosar, Light-Sensitive Systems, J. Wiley-Verlag 1965, S. 233 beschrieben.

Weiter ist bekannt, Resorcin und seine Derivate, wie seine Monoalkylester, in Diazotypiematerialien zur Erzeugung rotbrauner Farbstoffe zu verwenden. Es wird insbesondere zur Herstellung von lichtempfindlichen Diazoschichten für Zwischenoriginale verwendet, weil es braune und sepiafarbige Bilder ergibt, die das für Zwischenkopien erforderliche Lichtabsorptionsvermögen aufweisen (DE-B 26 09 545, entsprechend FR-A 23 44 051, DE-C 965 865, entsprechend FR-A 949 872).

Es hat sich jedoch gezeigt, daß die genannten Hydroxyphenyle und -diphenyle zwar gute Braunkuppler sind, ihre Herstellung jedoch schwierig und umständlich ist und einen hohen technischen Aufwand erfordert wegen der zur Einführung der phenolischen Hydroxygruppen notwendigen Ätznatronschmelzen der zugrunde liegenden Benzolsulfonsäuren bei hoher Temperatur. (Winnacker, Küchler, Chem.Technologie, Hanser-Verlag, 1959, 2. Aufl., Bd. 3 I, S. 836). Besonders wegen der aufwendigen Herstellung ist die Verfügbarkeit über die genannten Hydroxydiphenyle nicht immer gewährleistet.

Aus DE-A 21 00 232 ist eine wäßrige Lösung bekannt, die einen Gehalt von etwa 0,35 bis etwa 99 Teilen an einem festen, harzartigen Material besitzt, das bei der Destillation von technischem Resorcin in einer Destillationskolonne als Rückstand verbleibt. Die Lösung wird zur Herstellung von Formsandgemischen, etwa in 5 Teilen Lösung je 100 Teile Sand verwendet.

Es war Aufgabe der Erfindung eine Substanz oder ein Gemisch von Substanzen zur Verfügung zu stellen, das als Braunkupplungskomponente in Diazotypiematerialien eingesetzt werden kann, das leicht verfügbar ist und möglichst vergleichbare oder gleich gute technische Eigenschaften aufweist wie die bekannten Braunkuppler.

Die Erfindung betrifft ein Gemisch überwiegend mehrkerniger polyfunktioneller Phenole, hergestellt durch Lösen des bei der Resorcinherstellung anfallenden Destillationsrückstands in polaren organischen Lösungsmitteln und/oder Wasser sowie Reinigung der erhaltenen Lösung durch Aktivkohlefiltration gegebenenfalls unter Zusatz von Filterhilfsmitteln

Vorzugsweise betrifft die Erfindung ein Gemisch, das unter Zusatz von 5 - 20 Gewichtsprozent Resorcin, bezogen auf den eingesetzten Destillationsrückstand hergestellt wurde.

Das erfindungsgemäße Gemisch enthält

a) Verbindungen der allgemeinen Formel I

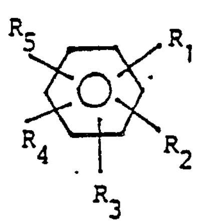

worin $R_1$ eine Hydroxy-, Niederalkyl- oder Hydroxyniederalkylgruppe
$R_2$ eine Hydroxy-, eine Mercapto- oder Hydroxy-niederalkylgruppe
$R_{3,4\ und\ 5}$ gleich oder verschieden sind und Wasserstoff oder Niederalkyl,
b) Verbindungen der allgemeinen Formel II

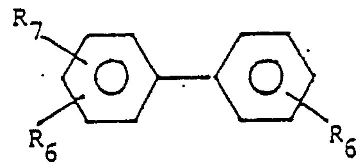

worin $R_6$ eine Hydroxygruppe und
$R_7$ Wasserstoff oder eine Hydroxygruppe und
c) Verbindungen der allgemeinen Formel III

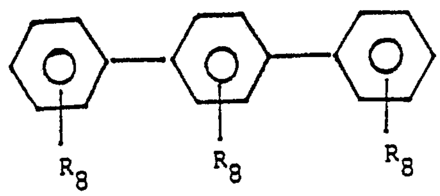

worin $R_8$ die Hydroxygruppe
bedeuten.

Der Anteil im Gemisch an Verbindungen nach der Formel I beträgt vorzugsweise etwa 0 bis 30, an Verbindungen der Formel II etwa 70 - 100 und der Anteil an Verbindungen der Formel III etwa 0 bis 10 Gewichtsprozent. Die Substituenten in den angeführten Formeln können sowohl in o-, m- oder p-Position zueinander stehen. Als Niederalkylgruppen sind ($C_1$ bis $C_2$)-Alkyl, insbesondere Methyl-Gruppen bevorzugt.

Wie eingangs bereits erwähnt, dient als Ausgangsmaterial für die Herstellung des erfindungsgemäßen Gemisches der Destillationsrückstand, der bei der Resorcinherstellung anfällt und bekannterweise z.B. als Formsandzusatz in Eisengießereien verwendet wird, zum anderen Teil aber als Abfall entsorgt werden muß.

Völlig überraschend kann man aus dem dunkel gefärbten Destillationsrückstand (Resorcinpech) ohne aufwendige Reinigungsmethoden oder Trennoperationen in einfacher Weise ein Gemisch gewinnen, das als Kupplungskomponente in Diazotypiematerialien geeignet ist und das die erwünschte braune Kupplerfarbe ergibt ohne den Pausengrund des Materials in unerwünschter Weise zu verfärben. Während man wie die üblicherweise als Kuppler eingesetzten Mono- und Polyhydroxydiphenyle bei ihrer Herstellung durch Umkristallisieren oder Destillieren reinigt, genügt bei dem erfindungsgemäßen Gemisch eine einfache Klärung der Lösungsfraktion mit Aktivkohle. Bei dieser Operation werden die höhermolekularen Bestandteile des Resorcinpechs als unlöslicher Rückstand abgetrennt. Außerdem wird die braune Lösung des Gemischs durch die Aktivkohle deutlich entfärbt, da die für die Farbe verantwortlichen Bestandteile an der Aktivkohle adsorbiert und zurückgehalten werden.

Die Herstellung des erfindungsgemäßen Gemisches erfolgt dadurch, daß man den bei der Resorcinherstellung anfallenden Destillationsrückstand in polaren organischen Lösungsmitteln und/oder Wasser löst und die erhaltene Lösung durch Aktivkohlefiltration reinigt.

Die Klärfiltration kann auch so erfolgen, daß man der Aktivkohle noch ein Filterhilfsmittel wie Kieselgur bei der Filtration zusetzt. Weiterhin kann die Klärfiltration zur Herstellung eines Kupplergemischs auch in der Weise durchgeführt werden, daß man dem Resorcinpech zunächst weitere Bestandteile des lichtempfindlichen Beschichtungsgemischs zufügt und anschließend erst filtriert.

Das erfindungsgemäße Gemisch wird vorzugsweise unter Zusatz von 5 - 20 Gewichtsprozent Resorcin, bezogen auf den eingesetzten Destillationsrückstand, hergestellt. Geeignete Lösungsmittel für das erfindungsgemäße Gemisch sind polare organische Lösungsmittel, wie aliphatische Alkohole, wie Propanol oder Isopropanol, Ethylenglykole, Ketone, Dimethylformamid und Dimethylsulfoxid und/oder Wasser oder deren Gemische. Wasser/Alkohol-Gemische werden bevorzugt verwendet.

Man löst das vorzugsweise höher konzentrierte Gemisch und kann nach der Aktivkohlefiltration die Lösung direkt als Kupplergemisch weiterverarbeiten. In diesem Fall werden vorzugsweise nur solche Lösungsmittel verwendet, die bei der Trocknung des Diazotypiematerials bezüglich Brennbarkeit und Toxizität unbedenklich sind. Bevorzugt wird für diesen Zweck ein Triethylenglykol-Wasser-Gemisch, vorzugsweise im Gewichtsverhältnis von 1:1.

Die Lösetemperatur kann zwischen 0 und 100° C liegen. Vorzugsweise liegt sie bei 20 - 50 °C. Man erreicht so eine möglichst kurze Lösezeit. Die Anwendung höherer Temperaturen, so wurde erkannt, führt

zu einem Harz, das man nur schwer in Lösung bringen kann.

Obwohl die Verarbeitung des erfindungsgemäßen Gemischs vorzugsweise in Lösung erfolgt, kann es auch als Feststoff in die Lichtpaus-Beschichtungslösung eingebracht werden. Bei einer derartigen Verarbeitung erfolgt die Klärfiltration zusammen mit anderen Schichtbestandteilen vorzugsweise auf der Stufe der fertigen Beschichtungslösung.

In einer besonders bevorzugten Ausführungsform wird das Gemisch so hergestellt, daß man Resorcinpech zusammen mit 5 - 20 Gewichtsprozent Resorcin (bezogen auf Resorcinpech) in einem Gemisch von Wasser und Triethylenglykol im Gewichtsverhältnis 1:1 löst. Ein so hergestelltes Gemisch läßt sich besonders einfach verarbeiten.

Das erfindungsgemäße Gemisch wird vorzugsweise als Kupplungskomponente zur Herstellung von lichtempfindlichen Diazotypiematerialien verwendet.

Durch die Erfindung wird erreicht, daß man aus für Abfall angesehenen oder nur noch in Formsanden verwendeten Massen auf einfache Weise, d.h. durch Lösen in einem umweltfreundlichen Lösungsmittel oder Lösemittelgemisch, Gemische von Verbindungen sowohl im Lösungszustand als auch fest zur Verfügung stellen kann, die als Kupplungskomponente für Brauneinstellungen in Diazotypiematerialien geeignet sind.

Diazotypiematerialien sind als Ein- oder Zweikomponentenmaterialien bekannt. Das erfindungsgemäße Gemisch wird als Kupplermischung der verbindungen nach den allgemeinen Formeln I, II, III bevorzugt in Zweikomponenten-Diazotypiematerialien verwendet. Diese enthalten neben den Kupplungskomponenten ein lichtempfindliches Diazoniumsalz, saure Stabilisatoren und andere übliche Zusätze.

Die Entwicklung erfolgt wie üblich nach bildweiser Belichtung mit einem alkalischen , vorzugsweise wäßrig-alkalischen Entwickler.

Als lichtempfindliche Substanzen werden übliche Dialkoxybenzoldiazoniumverbindungen eingesetzt. Im allgemeinen handelt es sich hierbei um Benzoldiazoniumverbindungen, die in der Position 4 eine tert. Aminogruppe, eine sekundäre Acylaminogruppe, eine Phenylgruppe oder eine veretherte Mercaptogruppe aufweisen und die in einer oder zwei der übrigen Stellungen durch ein Halogenatom, eine Alkyl-, Alkoxy-, Phenoxy- oder tert. Acylaminogruppe oder eine Kombination solcher Substituenten weiter substituiert sind.

Weitere Kupplungskomponenten können den erfindungsgemäßen Gemischen in den Beschichtungslösungen zur Farbtonstellung hinzugefügt werden, wie etwa: Dihydroxynaphthaline, Dihydroxynaphthalinmono- und -disulfonsäuren sowie deren Amide und substituierte Amide, α- und β-Hydroxynaphthoesäureamide und entsprechend substituierte Amide, Verbindungen mit aktiven Methylengruppen, wie Acetoacetyl- und Cyanoacetyl-Derivate oder Pyrazolon-Derivate.

Als Zusatz zu den Diazobeschichtungsmassen können bekannte Verbindungen verwendet werden, wie zum Beispiel Säurestabilisatoren, wie Citronensäure, Weinsäure, Borsäure, Sulfosalicylsäure, p-Toluolsulfosäure oder deren Gemische, Verbindungen zur Kontraststeigerung, wie Zinkchlorid, Aluminiumsulfat oder Nickelsulfat, Antioxydantien, wie Thioharnstoff oder Thioharnstoffderivate und Farbstoffe in kleiner Konzentration, wie Methylviolett oder Alizarinirisol zur Stabilisierung und Verbesserung des ausbelichteten Hintergrundes, Entwicklungsbeschleuniger, wie Glycerin, Glycerinmono-, di- und -triacetat, Harnstoff und alkylsubstituierte Harnstoffe, feinteiliges oder kolloidales Siliziumdioxid oder Aluminiumdioxid oder/und wäßrige Dispersionen oder kolloidale Lösungen organischer filmbildender Bindemittel, wie Polyvinylalkohol, Hydroxyethylcellulose, Methylcellulose oder latexartige Dispersionen von Polyacrylnitril oder Polymethylmethacrylat.

Bei Verwendung von Kunststoffolien als Schichtträger für das Diazotypiematerial ist es vorteilhaft, die Diazobeschichtungsmasse aus einem organischen Medium, das ein filmbildendes Bindemittel gelöst enthält, auf die Schichtträger aufzubringen. Geeignete filmbildende Bindemittel sind beispielsweise Celluloseether, wie Ethylcellulose, Celluloseester, wie Celluloseacetat, -acetopropionat, -butyrat, -acetobutyrat, Vinylpolymerisate, wie Polyvinylacetat, Polymethylmethacrylat, Mischpolymerisate von Alkylacrylaten und Acrylsäure oder Polyphenylenoxide oder Ethylenglykol/Isophthalsäure/Terephthalsäure-Terpolymerisate.

Die Mengenverhältnisse der einzelnen Komponenten der Diazotypiematerialien können die gleichen sein wie sie bisher zur Herstellung lichtempfindlicher Diazozusammensetzungen verwendeten wurden. Diese Verhältnisse sind allgemein bekannt, so z.B. aus Kosar, Light-Sensitive Systems, Wiley-verlag, New York, Seiten 292-296.

Als Schichtträger eignen sich alle üblichen opaken und transparenten Materialien, wie beschichtete oder unbeschichtete, opake oder transparente Papiere, Celluloseester, wie Cellulose-2-1/2-acetat und Cellulosetriacetat, Polyester, wie Polyethylenterephthalat, was bevorzugt ist, vinylpolymere, wie Polyvinylacetat oder Polystyrol sowie Polymere, wie Polyethylen oder Polypropylen

Die Verarbeitung des Diazotypiematerials erfolgt, wie üblich, durch bildmäßige Belichtung unter einer transparenten Vorlage mit einer an ultravioletter und kurzwelliger sichtbarer Strahlung reichen Lichtquelle,

beispielsweise mit einer Quecksilberhochdrucklampe oder einer Fluoreszenz-Leuchtstofflampe und durch anschließende Entwicklung, etwa mit feuchtem oder trockenem Ammoniakgas bei normaler oder erhöhter Temperatur oder alkalischen Entwicklerlösungen.

Die folgenden Beispiele sollen die Erfindung näher erläutern.

**Herstellungsbeispiele für das erfindungsgemäße Gemisch:**

A) Kupplergemisch in Isopropanol

10 g Resorcinpech wurden bei Raumtemperatur unter Rühren innerhalb von 16 Stunden in 40 g Isopropanol gelöst. Die braune Lösung wurde anschließend mit je 0,5 g Aktivkohle und Kieselgur versetzt, eine halbe Stunde gerührt und mit Stickstoff über ein Druckfilter gedrückt. Man erhielt 48 g 20 %ige hellbraune Kupplerlösung, die in dieser Form als Kupplungskomponente weiterverarbeitet wurde.

Wie eine qualitative Charakterisierung durch die Molmasse (Kapillar GC/MS ) sowie vergleichende quantitative Analysen durch Kapillar-Gaschromatographie und naßchemische Bestimmung der Hydroxygruppen durch Acetylierung zeigen, besteht Resorcinpech vorwiegend aus Dihydroxy-, Trihydroxy-diphenylen mit geringen Anteilen an Resorcin, Mercaptophenol, Alkyl- und Hydroxyalkyl substituierten Benzolen und Polyhydroxy-triphenylen, wie dies mit 10 %igen Lösungen in Ethylacetat gemäß obigen Untersuchungen ermittelt wurde, etwa in der folgenden Zusammensetzung:

**Tabelle:**

| % | Strukturen |
|---|---|
| 1 | 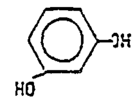 |
| 2 | 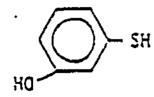 |
| 0,3 | 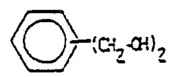 |
| 0,3 | 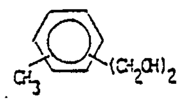 |
| 1,5 | 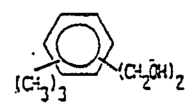 |
| 43,5 | 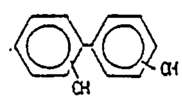 |
| 47 | 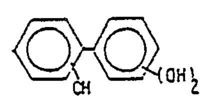 |
| 3 | 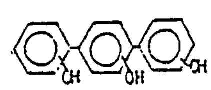 |

B) Kupplergemisch in Ethylenglykol

10 g Resorcinpech wurden bei 50° C unter Rühren innerhalb von 4 Stunden in 40 g Ethylenglykol gelöst. Die braune Lösung wurde anschließend mit je 1 g Aktivkohle und Kieselgur versetzt, eine halbe Stunde gerührt und mit Stickstoff über ein Druckfilter filtriert. Man erhielt 48 g einer 20 %igen hellbraunen Kupplerlösung, die in dieser Form weiterverarbeitet wurde.

C) Kupplergemisch mit erhöhtem Resorcinanteil in Wasser

10 g Resorcinpech und 1,5 g Resorcin wurden bei Raumtemperatur unter Rühren innerhalb von 45 Minuten in 46 ml Wasser gelöst. Anschließend gab man zu der braunen Lösung je 1 g Aktivkohle und Kieselgur und filtrierte über ein Druckfilter. Man erhielt 55 g einer 20 %igen hellbraunen Kupplerlösung.

D) Kupplergemisch mit erhöhtem Resorcinanteil in Wasser/Triethylenglykol

10 g Resorcinpech und 1,5 g Resorcin wurden bei 45° C unter Rühren innerhalb von 2,5 Stunden in 13,4 g Triethylenglykol und 13,4 g Wasser gelöst. Man gab je 1 g Aktivkohle und Kieselgur zu der braunen Lösung, rührte 30 Minuten und filtrierte über eine Nutsche. Man erhielt 37 g einer 30 %igen hellbraunen Kupplerlösung.

E) Kupplergemisch mit erhöhtem Resorcinanteil in Wasser/Triethylenglykol

23,8 g Resorcinpech wurden durch Chromatographie an Kieselgur (800 g) mit Cyclohexan/Ethylacetat (10 : 1) als Laufmittel von gefärbten und höhermolekularen Bestandteilen gereinigt. Nach Entfernen des Lösungsmittels erhielt man 18,0 g gelbbraunes Harz.

16,4 g dieses Harzes und 2,46 g Resorcin wurden bei Raumtemperatur unter Rühren innerhalb von 16 Stunden in 22 g Triethylenglykol und 22 g Wasser gelöst. Man erhielt 62,8 g einer 30 eigen hellbraunen Kupplerlösung, die in dieser Form als Kupplungskomponente für ein Zweikomponenten-Diazotypiematerial verwendet wurde.

F) Kupplergemisch mit erhöhtem Resorcinanteil in Wasser/Triethylenglykol

20,0 g Resorcinpech und 6,0 g Resorcin wurden bei Raumtemperatur unter Rühren innerhalb von 16 Stunden in 30,3 g Triethylenglykol und 30,3 g Wasser gelöst. Man gab je 1 g Aktivkohle und Kieselgur zu der braunen Lösung, rührte 30 Minuten und filtrierte über eine Nutsche. Man erhielt 85 g einer 30 %igen hellbraunen Kupplerlösung, die in dieser Form weiterverarbeitet wurde.

**Beispiel 1:**

Ein transparentes Pauspapier mit einem Flächengewicht von 80 g/m$^2$ wird einseitig mit Celluloseacetobutyrat versehen und danach die lackierte Seite sensibilisiert mit einer Lösung von:.

700 ml Isopropanol
100 ml Weichwasser
100 ml Ameisensäure
35 g Sulfosalicylsäure
200 ml Kupplerlösung aus Herstellungsbeispiel (H.B.) A (20 % in Isopropanol)
7 g 2-Hydroxynaphthalin-3-carbonsäure-N-(2′-hydroxyethylamid)
20 g 4-N-Pyrrolidino-3-methylbenzoldiazoniumsulfosalicylat

Das nach Trocknen auf diese Weise erhaltene lichtempfindliche Transparentpapier wird durch eine transparente Vorlage belichtet und mit Ammoniak entwickelt. An den Bildstellen entsteht ein tiefbrauner Farbton. Diese Kopie ist als Zwischenoriginal für weitere Pausen geeignet.

**Beispiel 2:**

Ein für das Diazotypieverfahren geeigneter Papierträger wird mit einem Vorstrich beschichtet. Dieser besteht aus einer wässrigen $SiO_2$-Suspension und einem Bindemittelsystem, wie eine Polyvinylacetatemulsion oder einer wässrigen Lösung von Natriumcaseinat. Der so beschichtete Papierträger wird mit einer Flüssigkeit mit folgenden Komponenten sensibilisiert:

200 g Citronensäure
150 g Coffein
500 g Thioharnstoff
250 g Kupplermischung H.B. B (20 % in Ethylenglykol)
110 g 2,7-Dihydroxy-3,6-naphthalindisulfonsäure
175 g 4-N,N-Dimethylaminobenzoldiazoniumsalicylat
50 g Zinkchlorid
2 g Saponin
10 000 cm$^3$ Wasser

Das getrocknete, sensibilisierte Material wird unter einer Vorlage belichtet und anschließend mittels Ammoniak entwickelt. An den Bildstellen entsteht ein tiefschwarzer Farbton.

**Beispiel 3:**

Ein für das Diazotypieverfahren geeigneter und mit einem Vorstrich versehener Papierträger wird mit folgenden Beschichtungslösungen sensibilisiert:

| | Lösung 1 | Lösung 2 |
|---|---|---|
| Citronensäure | 200 g | 200 g |
| Coffein | 150 g | 150 g |
| Thioharnstoff | 500 g | 500 g |
| Kupplermischung H.B. D(30 %ig $H_2O$/Triethylenglykol) | 170 g | - |
| Kupplermischung H.B. E (30 %ig $H_2O$/Triethylenglykol) | - | 170 g |
| 2,7-Dihydroxy-3,6-naphthalindisulfonsäure | 110 g | 110 g |
| 4-N,N-Dimethylaminobenzoldiazoniumsulfosalicylat | 175 g | 175 g |
| Zinkchlorid | 50 g | 50 g |
| Saponin | 2 g | 2 g |
| Wasser | 10 000 $cm^3$ | 10 000 $cm^3$ |

Das lichtempfindliche Material, das beim Sensibilisieren mit der Lösung I entsteht, wird mit dem lichtempfindlichen Material der Lösung II verglichen. Bezüglich der Lichtempfindlichkeit, dem Farbort des Volltons und des ausbelichteten Grundes und der Haltbarkeit sind keine Unterschiede feststellbar.

**Beispiel 4:**

Ein wie im Beispiel 2 vorbehandeltes Papier wird mit Flüssigkeiten folgender Zusammensetzung sensibilisiert:

| | Lösung I | Lösung II | Lösung III |
|---|---|---|---|
| Citronensäure | 200 g | 200 g | 200 g |
| Coffein | 150 g | 150 g | 150 g |
| Thioharnstoff | 500 g | 500 g | 500 g |
| Kupplerlösung H.B. B | 250 g | - | - |
| Kupplerlösung H.B. D | - | 165 g | - |
| Kupplerlösung H.B. F | - | - | 165 g |
| 2,7-Dihydroxy-3,6-naphthalindisulfonsäure | 110 g | 110 g | 110 g |
| 4-N,N-Dimethylaminobenzoldiazoniumsulfosalicylat | 175 g | 175 g | 175 g |
| Zinkchlorid | 500 g | 500 g | 500 g |
| Saponin | 2 g | 2 g | 2 g |
| Wasser | 10 000 g | 10 000 g | 10 000 g |

Die getrockneten, sensibilisierten Materialien werden unter einer Vorlage belichtet und mittels Ammoniak entwickelt. Die Volltöne der Materialien besitzen einen tiefschwarzen Farbton. Die Charakterisierung dieser Farbtöne mittels L, a, b- Werten zeigt, daß die Substitution der Lösung I durch die Lösung II bzw. durch die Lösung III eine Reduzierung des Blauanteils bewirkt.

| | Lösung I Vollton | Lösung II Vollton | LösungIII Vollton |
|---|---|---|---|
| L | 17,4 | 17,3 | 17,2 |
| a | + 2,3 | + 2,5 | + 2,4 |
| b | - 5,5 | - 4,8 | - 4,0 |

Hierbei geben der L-Wert die Helligkeit, der a-Wert den Rot-Grün-Anteil und der b-Wert den Gelb-Blau-Anteil des Farbtons an.

**Beispiel 5:**

Ein für das Diazotypieverfahren geeigneter und mit einem Vorstrich versehener Papierträger wird mit folgender Beschichtungslösung sensibilisiert:
50 g Citronensäure
350 g Naphthalin-1,3,6-trisulfonsäure (Natriumsalz)
60 g Aluminiumsulfat
160 g 2,5-Diethoxy-4-thio(4′-methylphenyl)benzoldiazoniumsulfat
10.000 g Wasser

Das sensibilisierte Material wird durch eine Vorlage belichtet und als Einkomponenten-Diazotypiematerial mit einem Flüssigentwickler folgender Zusammensetzung entwickelt:
35 g Natriumbenzoat
36 g Trinatriumcitrat
50 g Kupplermischung Bsp. C (20 %ige $H_2O$)
1.000 g Wasser

Im Bildbereich bildet sich ein schwarzblauer Azofarbstoff.

## Ansprüche

1. Gemisch überwiegend mehrkerniger polyfunktioneller Phenole, hergestellt durch Lösen des bei der Resorcinherstellung anfallenden Destillationsrückstands in polaren organischen Lösungsmitteln und/oder Wasser sowie Reinigung der erhaltenen Lösung durch Aktivkohlefiltration gegebenenfalls unter Zusatz von Filterhilfsmitteln.
2. Gemisch nach Anspruch 1 hergestellt unter Zusatz von 5 - 20 Gewichtsprozent Resorcin, bezogen auf den eingesetzten Destillationsrückstand.
3. Verwendung des Gemisches nach Anspruch 1 oder 2 als Kupplungskomponente zur Herstellung von lichtempfindlichen Diazotypiematerialien.
4. Verwendung des Gemisches nach Anspruch 3 in Zweikomponenten-Diazotypiematerialien.

Europäisches Patentamt

# EUROPÄISCHER RECHERCHENBERICHT

Nummer der Anmeldung

EP 90121784.4

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
| A | US - A - 4 503 267 (MARK S. PAVLIN) * Zusammenfassung * -- | 1 | C 07 C 39/15<br>C 07 C 37/70<br>G 03 C 1/52 |
| D,A | DE - A - 1 597 617 (KALLE AG) * Anspruch 1; Seite 13 * -- | 1,3,4 | |
| D,A | DE - C - 965 865 (GENERAL ANILINE & FILM) * Anspruch 1 * ---- | 1,3 | |
| | | | RECHERCHIERTE SACHGEBIETE (Int Cl 5) |
| | | | C 07 C 39/00<br>C 07 C 37/00<br>G 03 C |

Der vorliegende Recherchenbericht wurde für alle Patentansprüche erstellt.

| Recherchenort | Abschlußdatum der Recherche | Prüfer |
|---|---|---|
| WIEN | 22-02-1991 | REIF |

KATEGORIE DER GENANNTEN DOKUMENTEN

X : von besonderer Bedeutung allein betrachtet
Y : von besonderer Bedeutung in Verbindung mit einer anderen Veroffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veroffentlicht worden ist
D : in der Anmeldung angeführtes Dokument
L : aus andern Gründen angeführtes Dokument

& : Mitglied der gleichen Patentfamilie, übereinstimmendes Dokument